(19) Europäisches Patentamt
European Patent Office
Office européen des brevets

(11) Publication number: **0 002 937 A1**

(12) **EUROPEAN PATENT APPLICATION**

(21) Application number: **78300879.0**

(22) Date of filing: **21.12.78**

(51) Int. Cl.²: **C 07 D 221/04**, C 07 D 405/06, A 61 K 31/435 // C07D211/52, C07D211/70

(30) Priority: **27.12.77 US 864899**

(43) Date of publication of application: **11.07.79 Bulletin 79/14**

(84) Designated Contracting States: **DE GB IT NL SE**

(71) Applicant: **ELI LILLY AND COMPANY, 307, East McCarty Street, Indianapolis Indiana (US)**

(72) Inventor: **Zimmerman. Dennis Michael, 131, Thorncrest Drive, Mooresvllle Indiana 46158 (US)**

(74) Representative: **McVey, Kenneth William Henry et al, Erl Wood Manor, Windlesham Surrey, GU20 6PH (GB)**

(54) **Novel pyrindines and their preparation, formulations and use.**

(57) Trans-4a-phenyl and substituted phenyl 2,3,4,4a,5,6,7,7a-octahydro-1H-2-pyrindines of the formula

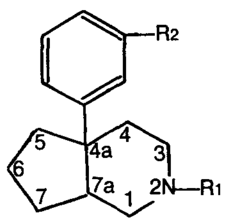

(I)

wherein:
$R_1$ is hydrogen, $C_1$–$C_8$ alkyl, $CH_2R_3$,

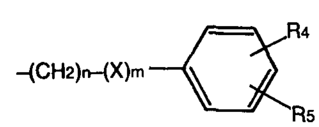

,

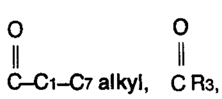

or

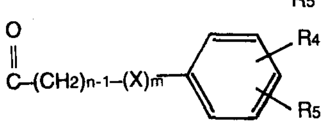

;

in which:
$R_3$ is $C_2$–$C_7$ alkenyl, $C_3$–$C_6$ cycloalkyl, furyl, or tetrahydrofuryl;
$R_4$ and $R_5$ independently are hydrogen, $C_1$–$C_3$ alkyl, or halogen;
n is 0, 1, 2, or 3;
m is 0 or 1, except that when m is o, n is other than O;
X is CO, CHOH, CH=CH, S, or O, except that when n is O, X is other than S or O;
$R_2$ is hydrogen, hydroxy, $C_1$–$C_3$ alkoxy, or $C_1$–$C_3$ alkanoyloxy; and
the non-toxic pharmaceutically acceptable acid addition salts thereof, are useful as analgesic agents having mixed agonist and antagonist properties. The compounds can be prepared by reacting a 4a-aryl-2-substituted-3,4,4a,5,6,7-hexahydro-2-pyrindine with hydrogen and platinum oxide.

EP 0 002 937 A1

0002937

# NOVEL PYRINDINES AND THEIR PREPARATION, FORMULATIONS AND USE

This invention relates to a novel group of pyrindines.

In recent years, much effort has been devoted to the synthesis of drugs, i.e. analgesics, capable of relieving the sensation of pain. Several of the currently available analgesics are limited in their use due to various undesirable side effects which frequently accompany their continued use. Such side effects include addiction and allergy. Illustrative of new analgesic drugs which have recently been discovered are the decahydroisoquinolines, particularly the 4a-aryl-trans-decahydroisoquinolines which are described in Belgian Patent No. 802,557.

The present invention relates to a group of trans-4a-aryl-2-substituted-octahydro-1H-2-pyrindines. Such compounds are somewhat structurally related to the aforementioned isoquinoline derivatives; however, the compounds of formula (I) below have not heretofore been synthetically available. Simple unsubstituted pyrindine analogs are known in the literature. Volodina et al., for example, prepared certain octahydro-2-pyrindines, none of which were substituted at the 4a-position; Dokl, Akad. Nauk USSR 173(2), 342-5 (1967) cf. C.A. Vol. 67, 6034(1967). Similarly, Prochazka et al. prepared a trans-octahydro-2-

pyrindine lacking a 4a-substituent, Coll. Czech. Chem. Commun., 31(9), 3824-8(1966), Cf. C.A. Vol. 65, 13651 (1966). Recently, Zimmerman prepared the cis-4a-aryl-2-substituted-octahydro-1H-2-pyrindines which are analgesics and are disclosed in Belgian Patent No. 860,314.

This invention provides trans-4a-phenyl and substituted phenyl 2,3,4,4a,5,6,7,7a-octahydro-1H-2-pyrindines not heretofore known or available, and intermediates useful in their preparation.

This invention relates to new bicylic compounds characterized as being octahydro-1H-2-pyrindines, alternatively referred to as hexahydro-1H-cyclopenta[c]pyridines. Specifically, the invention provides trans-4a-aryl-2-substituted-2,3,4,4a,5,6,7,7a-octahydro-1H-2-pyrindines of the general formula

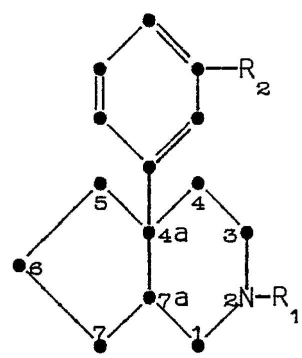

(I)

wherein: $R_1$ is hydrogen, $C_1$-$C_8$ alkyl, $CH_2R_3$,

$-(CH_2)_n-(X)_m-$ 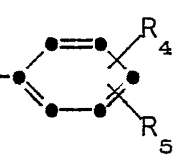 , $\overset{O}{\overset{\|}{C}}-C_1-C_7$ alkyl, $\overset{O}{\overset{\|}{C}}R_3$, or

$\overset{O}{\overset{\|}{C}}-(CH_2)_{n-1}-(X)_m-$ 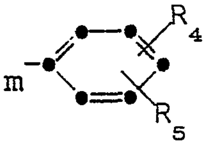 ; in which $R_3$

is $C_2$-$C_7$ alkenyl, $C_3$-$C_6$ cycloalkyl, furyl, or tetrahydrofuryl; $R_4$ and $R_5$ independently are hydrogen, $C_1$-$C_3$ alkyl, or halogen; n is 0, 1, 2, or 3; m is 0 or 1, except that when m is 0, n is other than 0; X is CO, CHOH, CH=CH, S, or O, except that when n is O, X is other than S or O; $R_2$ is hydrogen, hydroxy, $C_1$-$C_3$ alkoxy, or $C_1$-$C_3$ alkanoyloxy; and the non-toxic pharmaceutically acceptable acid addition salts thereof.

Additionally encompossed within the scope of formula (I) are intermediate compounds having the above formula wherein $R_1$ is $\overset{O}{\overset{\|}{C}}-C_1-C_7$ alkyl, $\overset{O}{\overset{\|}{C}}R_3$ in which $R_3$ has the above defined meaning,

$\overset{O}{\overset{\|}{C}}-(CH_2)_{n-1}-(X)_m$— 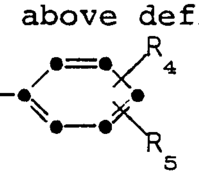 in which n, m, X, $R_4$ and $R_5$ have the above defined meaning.

A preferred group of compounds of formula (I) wherein $R_1$ is $C_1$-$C_8$ alkyl or $CH_2R_3$ in which $R_3$ is $C_2$-$C_7$ alkenyl or $C_3$-$C_6$ cycloalkyl. A more preferred group of compounds within this latter preferred group are those of formula (I) wherein $R_2$ is hydroxy methoxy. An especially preferred group of intermediate compounds are those of formula (I) wherein $R_1$ is hydrogen.

The process for preparing the trans-compounds of the general formula

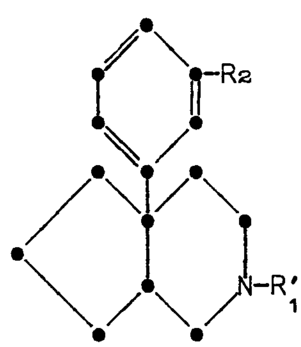

(II)

wherein:

$R_1'$ is hydrogen, $C_1$-$C_8$ alkyl, $CH_2R_3$, or $-(CH_2)_n-(X)_m-$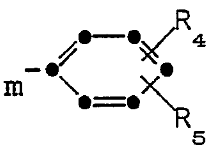 in which:

$R_3$ is $C_2$-$C_7$ alkenyl, $C_3$-$C_6$ cycloalkyl, furyl, or tetrahydrofuryl;

$R_4$ and $R_5$ independently are hydrogen, $C_1$-$C_3$ alkyl, or halogen;

n is 0, 1, 2, or 3;

m is 0 or 1, except that when m is 0, n is other than 0;

X is CO, CHOH, CH=CH, S, or O, except that when n is 0, X is other than S or O;

$R_2$ is hydrogen, hydroxy, $C_1$-$C_3$ alkoxy, or $C_1$-$C_3$ alkanoyloxy; and

the pharmaceutically acceptable acid addition salts thereof;

which comprises reacting a compound of the general formula

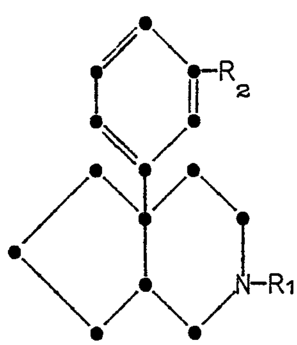

(III)

wherein:

$R_1$ is hydrogen, $C_1$-$C_8$ alkyl, $CH_2R_3'$, $-(CH_2)_n-(X)_m-$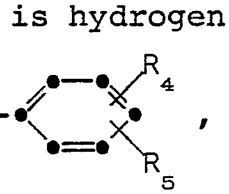, $\overset{O}{\overset{\|}{C}}$-$C_1$-$C_7$ alkyl, $\overset{O}{\overset{\|}{C}}R_3'$, or

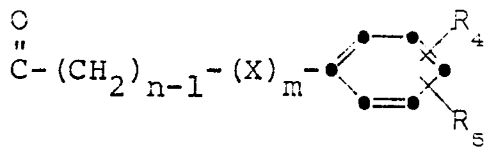

in which:

$R_3'$ is $C_3$-$C_6$ cycloallsyl, furyl, or tetrahydrofuryl;

$R_4$ and $R_5$ independently are hydrogen, $C_1$-$C_3$ alkyl, or halogen;

n is 0, 1, 2, or 3;

m is 0 or 1, except that when m is 0, n is other than 0;

X is CO, CHOH, CH=CH, S, or O, except that when n is 0, X is other than S or O;

$R_2$ is hydrogen, hydroxy, $C_1$-$C_3$ alkoxy, or $C_1$-$C_3$ alkanoyloxy;

with hydrogen and platinium oxide, followed by alkylation when $R_1$ is hydrogen to obtain a compound of formula (II) in which $R_1'$ is defined as before, optionally de-etherifying when $R_2$ is $C_1$-$C_3$ alkoxy to obtain a compound of formula (II) in which $R_2$ is hydroxy, and optionally acylating a compound of formula (II) in which $R_2$ is hydroxy to obtain a compound of formula (II) in which $R_2$ is $C_1$-$C_3$ alkanoyloxy, and, when desired, forming the pharmaceutically acceptable acid addition salt thereof by conventional means.

As used throughout the present specification and in the appended claims, the term "$C_1$-$C_8$ alkyl" refers to both straight and branched chains of eight carbon atoms or less. Examples of typical $C_1$-$C_8$ alkyl groups include methyl, ethyl, propyl, butyl, isopropyl, isobutyl, pentyl, 3-methylpentyl, 1,2-dimethylpentyl, 2-methylbutyl, 3-ethylpentyl, n-octyl, 2-methylheptyl, isoheptyl, 3-ethylhexyl, 1,3,3-trimethylpentyl, and related groups.

The term "$CH_2R_3$, in which $R_3$ is $C_2$-$C_7$ alkenyl" refers to both straight and branched alkenyl groups having eight or less carbon atoms, including groups such as allyl, 3-butenyl, 2-pentenyl, 3-pentenyl, 2-methyl-2-butenyl, 3-methyl-3-pentenyl, 3-isohexenyl, 2-ethyl-3-butenyl, 4-hexenyl, 3-methyl-2-pentenyl, 3-octenyl, 2-isooctenyl, 2-isopropyl-3-butenyl, 2,3-dimethyl-2-butenyl, 5-heptenyl, 6-octenyl, 2-methyl-3-heptenyl, and related alkenyl groups.

Additionally included within the definition of $R_1$ in formula (I) is the group represented by $CH_2R_3$ in which $R_3$ is $C_3$-$C_6$ cycloalkyl. Such groups include cyclopropylmethyl, cyclobutylmethyl, cyclopentylmethyl and cyclohexylmethyl. $R_1$ can also represent groups such as 2-tetrahydrofurylmethyl, 3-tetrahydrofurylmethyl, and 3-furylmethyl.

In formula (I), $R_1$ can also be a group of the formula $-(CH_2)_n-(X)_m$ 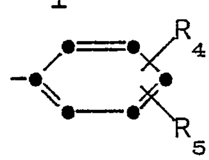 in which n is 0, 1, 2, or 3, m is 0 or 1, except that when m is 0, n is other than 0; X is CO, CHOH, CH=CH, S or O, except that when n is O, X is other than S or O; and $R_4$ and $R_5$ independently are hydrogen, $C_1$-$C_3$ alkyl, or halogen. In such formula, the term "$C_1$-$C_3$ alkyl" includes methyl, ethyl and propyl. "Halogen" refers to fluorine,

chlorine, bromine and iodine. Examples of typical $R_1$ groups represented by this partial formula include benzyl, 2-phenylethyl, 3-phenylpropyl, 3-methylbenzyl, 4-chlorobenzyl, 2,4-dibromobenzyl, 2-(2-methyl-5-ethylphenyl)ethyl, 3-(4-isopropylphenyl)propyl, benzoylmethyl, benzoylethyl, 4-iodobenzoylmethyl, 2-methyl-4-chlorobenzoylmethyl, 2-phenyl-2-hydroxyethyl, 3-phenyl-3-hydroxypropyl, 2-(4-fluorophenyl)-2-hydroxyethyl, phenoxymethyl, 3,5-diethylphenoxymethyl, 3-phenylthiopropyl, 2-methylphenylthiomethyl, 3,5-dichlorophenylthiomethyl, 3-chloro-5-bromophenylthiomethyl, and related groups.

When $R_1$ in formula (I) is hydrogen, the unsubstituted 2-position can be further reacted by acylation and reduction (either when the $\Delta^{1,7a}$-bond is reduced or separately with Zn/acetic acid or catalytic hydrogenation), or alkylation (particularly to obtain the group where $R_1$ is $CH_2R_3$ in which $R_3$ is $C_2$-$C_7$ alkenyl).

Compounds of formula (I) [as indicated by formula (II)] which are thus readily provided by reducing the $\Delta^{1,7a}$-bond of a 4a-aryl-2-substituted-3,4,4a,5,6,7-hexahydro-2-pyrindine of formula (III) according to the above-described procedures include, among others:

4a-phenyl-2,3,4,4a,5,6,7,7a-octahydro-1H-2-pyrindine;

4a-(3-methoxyphenyl)-2,3,4,4a,5,6,7,7a-octahydro-1H-2-pyrindine;

4a-(3-ethoxyphenyl)-2-methyl-2,3,4,4a,5,6,7,7a-octahydro-1H-2-pyrindine;

4a-phenyl-2-ethyl-2,3,4,4a,5,6,7,7a-octahydro-1H-2-pyrindine;

4a-(3-isopropoxyphenyl)-2-benzyl-2,3,4,4a,5,6,7,7a-octahydro-1H-2-pyrindine;

4a-phenyl-2-isobutyl-2,3,4,4a,5,6,7,7a-octahydro-1H-2-pyrindine;

4a-(3-methoxyphenyl)-2-(4-ethylhexyl)-2,3,4,4a,5,6,7,7a-octahydro-1H-2-pyrindine; and

4a-(3-ethoxyphenyl)-2-(3-chlorobenzyl)-2,3,4,4a,5,6,7,7a-octahydro-1H-2-pyrindine.

As was hereinbefore noted, very important intermediates for preparing all of the pyrindine derivatives of formula (I) are the 2-unsubstituted pyrindine derivatives, those in which $R_1$ in formula (II) is hydrogen. Such compounds can be readily alkylated or acylated at the 2-position to provide pharmacologically active octahydropyrindines of formula (I), or in the case of the N-acylated derivatives, to provide intermediates which are easily converted to the active analgesics of formula (I). It is therefore often desirable to prepare, according to the above-described processes, 4a-aryl-2-substituted-2,3,4,4a,5,6,7,7a-octahydro-1H-2-pyrindines in which the 2-substituent is readily removable so as to provide the corresponding 2-unsubstituted octahydropyrindine derivatives. The N-methyl and N-benzyl groups are readily cleavable to afford the corresponding 2-unsubstituted pyrindine derivative. The 2-methyl pyrindine derivatives can be reacted with a haloformate ester such as phenyl chloroformate or ethyl chloroformate to afford the corresponding carbamate at the pyrindine 2-position. Such carbamate

is then reacted with an aqueous base such as sodium hydroxide to effect cleavage of the 2-carbamate moiety and thus provide the corresponding 2-unsubstituted pyrindine derivative. Such method for the cleavage of an N-methyl group is that of Abel-Monen and Portoghese as described in J. Med. Chem., 15, 208(1972).

Similarly, the aforementioned 4a-aryl-2-benzyl-2,3,4,4a,5,6,7,7a-octahydro-1H-2-pyrindines are readily converted to the corresponding 2-unsubstituted pyrindine derivative by simple debenzylation. Such debenzylation may be achieved by catalytic hydrogention, utilizing for instance a catalyst such as five percent palladium suspended on carbon. Such debenzylation reactions are quite general for preparing secondary amines and are described in detail by Hartung and Simonoff, Org. Reactions, 7, 277(1953), and by Loenard and Fuji, J. Amer. Chem. Soc., 85, 3719 (1963).

As can readily be seen from the foregoing discussion, the following representative 2-unsubstituted pyrindine derivatives, formula (II) where $R_1$ is hydrogen, are also important intermediates for the preparation of the pyrindines of formula (I).

4a-phenyl-2,3,4,4a,5,6,7,7a-octahydro-1H-2-pyrindine;

4a-(3-methoxyphenyl)-2,3,4,4a,5,6,7,7a-octahydro-1H-2-pyrindine;

4a-(3-ethoxyphenyl)-2,3,4,4a,5,6,7,7a-octahydro-1H-2-pyrindine; and

4a-(3-isopropoxyphenyl)-2,3,4,4a,5,6,7,7a-octahydro-1H-2-pyrindine.

The 4a-aryl-2-unsubstituted-2,3,4,4a,5,6,7,7a-octahydro-1H-2-pyrindines thus prepared can be alkylated by normal procedures to provide pharmacologically active 2-substituted pyrindine derivatives, or can be acylated to provide intermediates which are readily converted to active analgesic drugs. For example, a 4a-aryl-2,3,4,4a,5,6,7,7a-octahydro-1H-2-pyrindine can be alkylated at the 2-position by reaction with essentially any reactive derivative of an alkyl group. Such alkylating agents are compounds of the formula $R_1$-Z in which $R_1$ is as defined hereinabove and Z is any of a number of groups commonly referred to as good leaving groups. Groups most commonly known as good leaving groups include the halogens, particularly chlorine, bromine and iodine, _para_-toluenesulfonyl (tosyl), phenylsulfonyl, methanesulfonyl (mesyl), _para_-bromophenylsulfonyl (brosyl), and azido. It will be noted that when reference is made herein to an alkylating agent having the formula $R_1$-Z, it is intended that the alkyl portion of such alkylating agent can be derivatized, for instance by unsaturated substituents, aryl substituents, and cycloalkyl substituents. The term "alkylating agent having the formula $R_1$-Z" thus includes compounds such as methyl chloride, ethyl bromide, 5-methylheptyltosylate, allyl bromide, 4-hexenyl iodide, 3-ethyl-4-pentenyl brosylate, cyclopropylmethyl chloride, cyclobutylmethyl iodide, cyclohexylmethyl mesylate, 3-tetrahydrofurylmethyl bromide, 2-furylmethyl azide, 2-phenylethyl chloride, 3-benzoylpropyl bromide, 2-(3-chlorophenylthio)ethyl azide, phenoxymethyl bromide, 3-isopropylphenylthiomethyl bromide, and related groups.

Thus, a 4a-aryl-2,3,4,4a,5,6,7,7a-octahydro-1H-2-pyrindine can be reacted with an alkylating agent to provide the corresponding 4a-aryl-2-substituted-2,3,4,4a,5,6,7,7a-octahydro-1H-2-pyrindine. Such alkylation reaction is quite general and can be accomplished by reacting the appropriate 4a-aryl-octahydro-1H-2-pyrindine with the appropriate alkylating agent, preferably in an unreactive organic solvent. The alkylating agent typically is utilized in excess amounts, for instance from about 0.5 to about 2.0 molar excess relative to the pyrindine derivative. Unreactive organic solvents commonly utilized in the reaction include ethers such as diethyl ether, dioxane, tetrahydrofuran, as well as solvents such as benzene, dichloromethane, dimethylformamide, dimethyl sulfoxide, nitromethane, and hexamethylphosphortriamide. A base is preferably incorporated in the alkylation reaction to act as an acid scavenger since the reaction of the pyrindine derivative and the alkylating agent generally is accompanied by the formation of an acid such as hydrochloric acid or para-toluenesulfonic acid which may act to tie up any unreacted 2-pyrindine derivative as a salt. Bases commonly utilized as acid scavengers in such reaction include sodium bicarbonate, potassium carbonate, sodium hydroxide, triethylamine, and pyridine. Typically, about one equivalent amount of

base is employed; however, excessive amounts can be incorporated if desired. The alkylation reaction normally is carried out at an elevated temperature ranging from about 50°C. to 200°C., and at such temperature, the reaction normally is substantially complete within about 1 to 10 hours; however, longer reaction times are not detrimental and can be used if desired. The product typically is recovered by simply adding water to the reaction mixture and then extracting the product therefrom into a water-immiscible organic solvent such as benzene, ethyl acetate, dichloromethane, diethyl ether, chloroform, or related solvents. Upon removal of the solvent from such extracts, for instance by evaporation under reduced pressure, there is obtained the product 4a-aryl-2-substituted-2,3,4,4a,5,6,7,7a-octahydro-1H-2-pyrindine, which compound exists either as an oil or as a solid at room temperature. The product so formed can be further purified if desired by standard procedures including chromatography, crystallization, distillation, or alternatively such pyrindine product can be converted to an acid addition salt by reaction with an inorganic or organic acid. Such salts routinely are highly crystalline solids and are readily recrystallized to provide a solid salt of high purity. If desired such salt can then be treated with a base such as sodium hydroxide or potassium carbonate, thereby cleaving the salt to provide the purified 4a-aryl-2-substituted-2,3,4,4a,5,6,7,7a-octahydro-1H-2-pyrindine as a free base.

As herebefore indicated, the 2-unsubstituted pyrindine derivatives, namely the 4a-aryl-octahydro-1H-2-pyrindines, can be converted to a 2-substituted pyrindine derivative which is either a pharmacologically useful agent per se, or one which can be readily converted to a pharmacologically useful agent. For example, reaction of a 4a-aryl-2,3,4,4a,5,6,7,7a-octahydro-1H-2-pyrindine with an alkylating agent such as 2-benzoylethyl iodide provides the corresponding 4a-aryl-2-(2-benzoylethyl)-2,3,4,4a,5,6,7,7a-octahydro-1H-2-pyrindine, an active analgesic. If desired, such compound can be reduced at the benzoyl carbonyl moiety, for instance by reaction with a reducing agent such as lithium aluminum hydride, to afford the corresponding 4a-aryl-2-(3-hydroxy-3-phenyl)propyl-2,3,4,4a,5,6,7,7a-octahydro-1H-2-pyrindine, also a useful analgesic agent. Additionally, a 2-unsubstituted pyrindine derivative can be acylated with any of a number of acylating agents to provide an N-acylated pyrindine derivative, a compound of formula (I) wherein $R_1$ is

$\overset{O}{\overset{\|}{C}}-C_1-C_7$ alkyl, $\overset{O}{\overset{\|}{C}}-R_3$, and $\overset{O}{\overset{\|}{C}}-(CH_2)_{n-1}-(X)_m-$ 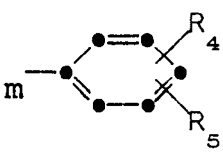 .

Such N-acylated pyrindines, upon reduction of the carbonyl moiety, provide 2-substituted pyrindine derivatives of formula (I) which are active analgesics. Examples of commonly used acylating agents include acetyl chloride, pentanoylchloride, 4-hexenoyl chloride, cyclobutylformyl bromide, 2-(tetrahydrofuryl)formyl chloride, benzoyl bromide, phenoxyacetyl iodide, 3,4-dimethylphenylacetyl chloride, 3-(2-fluorophenyl)propionyl chloride, phenylthioacetyl bromide, 4-phenyl-3-butenoyl chloride, acetic anhydride, and hexanoic anhydride. The acylation of the 2-unsubstituted pyrindine derivative with an acylating agent such as the aforementioned is carried out by reacting approximately equimolar quantities of the pyrindine derivative and the acylating agent in an unreactive organic solvent such as dichloromethane, ethanol, or tetrahydrofuran. The reaction typically utilizes a base such as sodium bicarbonate, potassium carbonate, or propylene oxide to serve as an acid scavenger. The reaction is best carried out at a temperature of about -20°C. to about 30°C., and generally is complete within 1 to 8 hours. The product, for example a 4a-aryl-2-acylated-2,3,4,4a,5,6,7,7a-octahydro-1H-2-pyrindine, is readily isolated by simply removing the reaction solvent by evaporation. The product so formed normally is not purified further, but rather is reduced immediately to provide a 4a-aryl-2-substituted-2,3,4,4a,5,6,7,7a-octahydro-1H-2-pyrindine of formula (I). Such reduction of the N-acyl carbonyl group can be accomplished by reaction of the acylated pyrindine derivative with a reducing agent such as lithium aluminum hydride or by catalytic hydrogenation or upon reduction of the $\Delta^{1,7a}$-bond with platinium oxide and hydrogen.

It will additionally be recognized that still other modifications can be made on certain of

the 4a-aryl-2-substituted-2,3,4,4a,5,6,7,7a-octahydro-1H-2-pyrindines of formula (I). For example, while a 4a-aryl pyrindine derivative wherein the aryl group is a 3-hydroxyphenyl moiety can be prepared by starting with a 2-(3-hydroxyphenyl)-2-ethoxycarbonylmethyl-cyclohexanone and modifying such compound according to the various processes discussed hereinabove, it might be preferable to prepare a 4a-(3-methoxyphenyl)-2-substituted-2,3,4,4a,5,6,7,7a-octahydro-1H-2-pyrindine, and then convert the 3-methoxy group of such 4a-aryl substituent to a hydroxy group. Such conversion is readily accomplished by reacting a 4a-(3-methoxyphenyl)-pyrindine derivative with hydrobromic acid in acetic acid. Such reaction is quite general for the conversion of a methoxyphenyl group to a hydroxyphenyl group. The hydroxy group of such 4a-(3-hydroxyphenyl)pyrindines can, if desired, be acylated with common $C_1$-$C_3$ alkanoyl acylating agents, for instance acetyl chloride or propionyl anhydride, thereby providing the corresponding 4a-(3-alkanoyloxyphenyl)pyrindine derivatives.

As hereinbefore pointed out, the 4a-aryl-2-substituted-octahydro-1H-2-pyrindine derivatives of formula (I) can be reacted with an organic or inorganic acid so as to provide a crystalline salt which can be purified by crystallization, and which then can be converted back to the pyrindine free base by treatment with a suitable base such as sodium hydroxide. Certain of the acid addition salts are

encompassed within the scope of formula (I). Specifically, there are included herein the non-toxic pharmaceutically acceptable acid addition salts of the pyrindine bases which are described hereinabove. Such non-toxic pharmaceutically acceptable acid addition salts are prepared by reacting a 4a-aryl-2-substituted-octahydro-1H-2-pyrindine of formula (I) with an organic or an inorganic acid. Acids commonly used to prepare the pharmaceutically acceptable acid addition salts of formula (I) include the hydrogen halide acids such as hydrogen chloride, hydrogen bromide, and hydrogen iodide, as well as acids such as sulfuric, phosphoric, nitric, perchloric, phosphorous, nitrous, and related acids. Organic acids commonly used to prepare pharmaceutically acceptable acid addition salts of the pyrindines of formula (I) include acetic, propionic, *para*-toluenesulfonic, chloroacetic, maleic, tartaric, succinic, oxalic, citric, lactic, palmitic, stearic, benzoic, and related acids. The pharmaceutically acceptable acid addition salts of formula (I) can be conveniently prepared by simply dissolving a 4a-aryl-2-substituted-octahydro-1H-2-pyrindine in a suitable solvent such as diethyl ether, ethyl acetate, acetone, or ethanol, and adding to such solution either an equivalent amount or an excess of a suitable acid. The salt so formed normally crystallizes out of solution and can be recovered by filtration, and is accordingly ready for use as a pharmacological agent, or can be further purified by recrystallization from common solvents such as acetone and methanol.

The following list of trans-4a-aryl-2-substituted-2,3,4,4a,5,6,7,7a-octahydro-1H-2-pyrindines is representative of the compounds falling within the scope of formula (I).

4a-phenyl-2-(3-ethylpentyl)-2,3,4,4a,5,6,7,7a-octahydro-1H-2-pyrindine;

4a-(3-methoxyphenyl)-2-(n-octyl)-2,3,4,4a,5,6,7,7a-octahydro-1H-2-pyrindinium bromide;

4a-(3-hydroxyphenyl)-2-(2-propenyl)-2,3,4,4a,5,6,7,7a-octahydro-1H-2-pyrindine;

4a-(3-propoxyphenyl)-2-(2,3-dimethyl-4-hexenyl)-2,3,4,4a,5,6,7,7a-octahydro-1H-2-pyrindine;

4a-phenyl-2-(5-heptenyl)-2,3,4,4a,5,6,7,7a-octahydro-1H-2-pyrindinium acetate;

4a-(3-hydroxyphenyl)-2-cyclopentylmethyl-2,3,4,4a,5,6,7,7a-octahydro-1H-2-pyrindinium oxalate;

4a-(3-ethoxyphenyl)-2-(2-tetrahydrofurylmethyl)-2,3,4,4a,5,6,7,7a-octahydro-1H-2-pyrindine;

4a-phenyl-2-(2-phenoxyethyl)-2,3,4,4a,5,6,7,7a-octahydro-1H-2-pyrindine;

4a-(3-hydroxyphenyl)-2-(2-methylphenoxymethyl)-2,3,4,4a,5,6,7,7a-octahydro-1H-2-pyrindinium succinate;

4a-(3-methoxyphenyl)-2-(3,5-dichlorobenzoyl-methyl)-2,3,4,4a,5,6,7,7a-octahydro-1H-2-pyrindine;

4a-(3-ethoxyphenyl)-2-[3-(3-methyl-4-bromophenyl)-3-hydroxy]propyl-2,3,4,4a,5,6,7,7a-octahydro-1H-2-pyrindinium iodide;

4a-phenyl-2-[3-(2-ethyl-6-methylphenylthio)-propyl]-2,3,4,4a,5,6,7,7a-octahydro-1H-2-pyrindinium perchlorate;

4a-(3-hydroxyphenyl)-2-[2-(3,4-dibromophenyl)-2-hydroxy]ethyl-2,3,4,4a,5,6,7,7a-octahydro-1H-2-pyrindine;

4a-phenyl-2-(3-phenylthio)propyl-2,3,4,4a,5,6,7,7a-octahydro-1H-2-pyrindinium citrate;

4a-phenyl-2-[3-(2-isopropylphenyl)propyl-2,3,4,4a,5,6,7,7a-octahydro-1H-2-pyrindinium maleate;

4a-(3-ethoxyphenyl)-2-(2-phenyl-2-hydroxyethyl)-2,3,4,4a,5,6,7,7a-octahydro-1H-2-pyrindinium phosphate;

4a-phenyl-2-[2-(4-chlorophenyl)-2-hydroxyethyl]-2,3,4,4a,5,6,7,7a-octahydro-1H-2-pyrindinium methanesulfonate;

4a-(3-hydroxyphenyl)-2-[3-(2-chloro-3-bromophenyl)-3-hydroxypropyl]-2,3,4,4a,5,6,7,7a-octahydro-1H-2-pyrindine;

4a-(3-propoxyphenyl)-2-(2-ethylbenzoylethyl)-2,3,4,4a,5,6,7,7a-octahydro-1H-2-pyrindinium chloride;

4a-(3-ethoxyphenyl)-2-[3-(2-chlorophenylthio)propyl]-2,3,4,4a,5,6,7,7a-octahydro-1H-2-pyrindine;

4a-phenyl-2-[3-(2-ethyl-5-bromophenyl)-propyl]-2,3,4,4a,5,6,7,7a-octahydro-1H-2-pyrindine; and

4a-(3-hydroxyphenyl)-2-[2-(3,5-diethylphenoxy)ethyl]-2,3,4,4a,5,6,7,7a-octahydro-1H-2-pyrindinium stearate.

It will be noted that the compounds of formula (I) have two asymmetric centers, namely the 4a position and the 7a position. This invention comprehends both separated isomers and racemic mixtures of such isomers which are useful pharmacologically as analgesic agonist or antogonist drugs.

However, only the trans-isomers of formula (I) are intended thereby, namely, such that the 4a-aryl group is oriented on the opposite side of the plane of the molecule from the 7a-hydrogen atom. This invention accordingly comprehends the pharmacologically active individual optically-active trans-isomers, in addition to the racemic mixture of trans-isomers. Such racemic pair of trans-octahydropyrindines can be separated into its component stereoisomers by procedures well known in the art. In the event that all useful pharmacologic activity resides in one sterioisomer, the dl-racemate is still useful in that it contains, as a constituent part, the pharmacologically active isomer.

The process for preparing the 4a,7a-trans isomers, formula (II), comprises catalytic hydrogenation of a 4a-aryl-hexahydropyrindine, formula (III), specifically a pyrindine having a double bond at the 1,7a-position. Such hydrogenation generally is carried out by reacting a 4a-aryl-2-alkyl-3,4,4a,5,6,7-hexahydro-2-pyrindine with hydrogen in the presence of a catalyst such as platinum oxide. The hydrogenation typically is carried out in a solvent such as methanol or ethanol, and routinely is complete within about one to eight hours when carried out at about 25°C. under a hydrogen pressure of about 2.74 x $10^6$ to about 5.48 x $10^6$ dynes/$cm^2$. The hydrogenation typically provides a mixture of the 1,7a-trans isomer and the 1,7a-cis isomer; however, the trans isomer generally predominates. Separation of

the isomers can be readily effected by salt formation and crystallization. For example the racemic mixture of octahydropyrindines can be converted to a suitable salt such as the pirate or maleate salt, and the *cis* racemate normally crystallizes first from solvents such as diethyl ether and diisopropyl ether, and can accordingly be separated from the *trans* by filtration. The *trans* racemate then can be recovered from the filtrate and purified by recrystallization.

The preparation of the 4a-aryl-octahydropyrindines provided by formula (I) requires starting materials, many of which are hitherto unknown and not readily available. For example, the above-described preferred method for preparing *trans*-4a-aryl-2-substituted-octahydro-1H-2-pyrindines requires the corresponding 4a-aryl-2-substituted-3,4,4a,5,6,7-hexahydro-2-pyrindines, *i.e.* $\Delta^{1,7a}$-hexahydropyrindines. Such compounds can be prepared by condensing phenyl lithium or a 3-substituted phenyl lithium with a 1-alkyl-4-piperidone to provide the corresponding 1-alkyl-4-phenyl or substituted phenyl-4-hydroxypiperidine. Dehydration of the 4-hydroxypiperidine derivative affords a 1-alkyl-4-aryl-1,2,3,6-tetrahydropyridine. The tetrahydropyridine derivative next is reacted with a propylene dihalide such as 3-chloropropylbromide to afford a 1-alkyl-4-aryl-4-(3-halopropyl)-1,2,3,4-tetrahydropyridine, which is then readily cyclized by reaction with sodium iodide in acetonitrile to provide the corresponding 4a-aryl-2-alkyl-3,4,4a,5,6,7-hexahydro-2-pyrindine.

Certain of the 4a-aryl-2-substituted octahydro-1H-2-pyrindines of formula (I) have found utility in the treatment of pain, and accordingly can be used to effect analgesia in a subject suffering from pain and in need of treatment. Additionally, the pyrindine derivatives of formula (I) have been found to possess both analgesic agonist and analgesic antagonist properties, and as such are capable of producing analgesia in a mammal while at the same time, because of the analgesic antagonist activity, having a greatly decreased incidence of addiction liability. Such ability of the compounds disclosed herein to cause analgesic agonist as well as analgesic antagonist effects in mammals is thus responsible for a decrease in any addictive properties of a particular drug caused by its opiate-like analgesic action. The compounds are thus particularly valuable since they produce analgesia with only minimal physical dependance liability. Certain of the compounds are additionally useful in combating the undesirable effects produced by opiates such as morphine.

The analgesic activity possessed by the compounds of formula (I) has been determined by testing such compounds in standard animal assays routinely used to measure analgesic action attributable to test compounds. Such assays include the mouse-writhing test and the rat tail jerk assay.

The mouse-writhing assay is carried out as follows:

Cox standard strain albino male mice, weighing 20-22 grams and fasted overnight, are used. Writhing, which is characterized by contraction of the abdominal musculature, extension of the hindlegs and rotation of the trunk is induced by the intraperitoneal administration of 55 mg/kg of acetic acid (0.55 percent). Each treatment group consists of 5 mice. The total number of writhes for the treatment group is determined in a 10-minute observation period starting 5 minutes after acetic acid administration. Control groups have totals of 200-350 writhes per observation period. Control and experimental groups are compared and a percent inhibition calculated as follows:

$$\text{\% Inhibition} = 100 - \left(\frac{\text{Experimental total}}{\text{Control total}}\right) \times 100$$

Test compounds are administered at 100 mg/kg by the oral route at 30, 90, and 180 minutes and by the subcutaneous route at 30 minutes before the intraperitoneal administration of acetic acid.

A second standard assay for analgesic activity is the rat tail jerk assay which is carried out as follows:

Female Sprague-Dawley rats weighing 70-80 grams that have been fasted overnight are used. Following treatment with either the administration vehicle or the test compound, the animal is placed in a plexiglass holder. A nichrome resistance wire is located between two V-shaped copper tubes that serve as a tail rest. The tail of the rat is placed in the tail rest and the nichrome wire is

heated by passing electric (AC) current through it. Radiant heat from the wire becomes aversive to a normal rat within 6-7 seconds and the rat attempts to "jerk" away from the heat. The elapsed time from the onset of the current in the wire to body "jerk" of the rat is recorded as the tail jerk reaction time. The average tail jerk reaction time of a drug-treated group is compared to the average reaction time of a vehicle-treated (control) group using Student's "t" test for statistical significance. These are usually 5 animals per treatment group. The mean reaction time $\pm$ S.E. of a typical control group is 6.85 $\pm$ 0.3 seconds.

In the mouse writhing assay, the following E.D.$_{50}$'s (dose (mg/kg) which decreases the number of writhing observations by 50 percent 30 minutes after administration compared to controls) were obtained for the compounds of formula (I) and are shown in Table I. Also, in the rat tail jerk assay, the following E.D.'s (effective dose (mg/kg) which causes a 2 second increase in reaction time determined after 30 minutes) were obtained for the compounds of formula (I) and are also shown in Table I.

Table I

| Example Number | Writhing (mg/kg) S.C. | Writhing (mg/kg) Oral | Rat Tail Jerk (sec) S.C. | Rat Tail Jerk (sec) Oral |
|---|---|---|---|---|
| 1 | 12.1 | 25.5 | 2.52* | 2.88* |
| 3 | 2.32 | 4.8 | -- | -- |
| 4 | 0.94 | 4.8 | 2.80Δ | 2.20Δ |

*increase in reaction time 10 mg/kg (30 min.) difference from control.

Δincrease in reaction time 0.5 mg/kg (30 min.) difference from control.

The 4a-aryl-2-substituted-2,3,4,4a,5,6,7,7a-octahydro-1H-2-pyrindines of formula (I) are thus useful in producing analgesia in mammals such as humans. Such compounds can be administered to a mammal by either the oral or the parenteral route. It generally is preferred to utilize a pharmaceutically acceptable acid addition salt of the pyrindine derivative when the dosage is by the oral route, since such salts are easily formulated for convenient oral administration. For example, one or more pharmacologically active compounds of formula (I) either as the free base or as a pharmaceutically acceptable acid addition salt, will be formulated for oral administration by admixing such compounds with any of a number of commonly used diluents, excipients, or carriers. Examples of such diluents and excipients commonly employed in pharmaceutical preparations include starch powder, sucrose, cellulose, magnesium stearate, lactose, calcium sulfate, sodium benzoate and related diluents. Such compositions can be molded into tablets or enclosed in telescoping gelatin capsules for convenient administration. If desired, the active compounds of formula (I) can additionally be combined with one or more other agents known to effect analgesia, such as caffeine, acetaminophen, and propoxyphene.

The active compounds of formula (I) can additionally be formulated as sterile aqueous or non-aqueous solutions, suspensions, and emulsions for convenient parenteral administration. Non-aqueous

vehicles commonly utilized in such formulations include propylene glycol, vegetable oils such as olive oil, as well as various organic esters such as ethyl oleate. Useful aqueous solutions for oral and parenteral administration include isotonic saline solution.

The precise dosage of active ingredient, that is the amount of one or more of the pharmacologically active 4a-aryl-2-substituted-octahydro-1H-2-pyrindines of formula (I) administered to a mammal, such as a human subject for example, may be varied over a relatively wide range, it being necessary that the formulations should constitute a proportion of one or more of the active ingredients of formula (I) such that a suitable dosage will be obtained. Such suitable dosage will depend on the particular therapeutic effect desired, on the particular route of administration being utilized, and on the duration of treatment, as well as the precise condition being treated. Typically the dosages of the active compounds of formula (I) will range from about 1.0 to about 25 mg./kg. of animal body weight per day, appropriately divided for administration from 1 to 4 times per day. Preferred oral dosages will generally range from about 2 to about 50 mg./kg.

In order to demonstrate more fully the scope of the compounds of formula (I) and their starting materials, the following examples are provided by way of illustration.

## STARTING MATERIALS

### Example A

A solution of 159 ml. of n-butyllithium in 100 ml. of hexane containing 47.7 g. of 3-methoxybromobenzene was stirred at -25°C. for twenty minutes and then was warmed to room temperature and stirred for one hour to provide 3-methoxyphenyl lithium. The reaction mixture was chilled to 10°C. and stirred while a solution of 50 g. of 1-methyl-4-piperidone in 100 ml. of diethyl ether was added dropwise over thirty minutes. Following complete addition, the reaction mixture was stirred for two hours, and then was diluted with 50 ml. of saturated aqueous sodium chloride solution. The solution was extracted several times with diethyl ether, and the ethereal extracts were combined and concentrated to dryness to provide 38 g. of 1-methyl-4-hydroxy-4-(3-methoxyphenyl)piperidine.

### Example B

To a stirred solution of 200 ml. of 50 g. of phosphorous pentoxide in methanesulfonic acid was added portionwise over four minutes 59 g. of 1-methyl-4-hydroxy-4-(3-hydroxyphenyl)piperidine. The reaction was exothermic and the temperature rose to 70°C. After complete addition of the piperidine derivative, the reaction mixture was added to 200 g. of ice, and the aqueous mixture was made alkaline by the addition of ammonium hydroxide. The alkaline mixture was extracted several times with diethyl ether, and the ethereal extracts were combined,

washed with water, dried, and the solvent was removed by evaporation under reduced pressure to provide 44.7 g. of the product as an oil. The oil thus formed was distilled to provide 1-methyl-4-(3-methoxyphenyl)-1,2,3,6-tetrahydropyridine, B.P. 123-138°C. at 0.1 torr.

Analysis Calc. for $C_{13}H_{17}NO$

Theory: C, 76.81; H, 8.43; N, 6.89.

Found: C, 76.52; H, 8.15; N, 6.67.

Example C

To a stirred cold (-5 to -10°C.) solution of 25 g. of 1-methyl-4-phenyl-1,2,3,6-tetrahydropyridine in 450 ml. of tetrahydrofuran was added dropwise over thirty minutes 90 ml. of 1.6 molar *n*-butyl lithium in hexane. Following complete addition, the solution was stirred for ten minutes at -10°C. and then cooled to -30°C. The cold solution next was added dropwise over twenty minutes to a stirred solution of 73.3 g. of 3-chloropropylbromide in 300 ml. of diethyl ether chilled to -50°C. Following complete addition, the reaction mixture was warmed to -20°C. and diluted with 500 ml. of saturated aqueous sodium chloride that had been chilled to 0°C. The organic layer was separated, washed with water, and the product was extracted therefrom into 1200 ml. of 1N hydrochloric acid. The aqueous acid layer was washed with diethyl ether and then was made alkaline by the dropwise addition of concentrated aqueous sodium hydroxide. The alkaline solution was extracted several times with diethyl ether, and the ethereal

extracts were combined, washed with water and dried. Evaporation of the solvent at 10°C. afforded an oil which was dissolved in 2500 ml. of acetonitrile containing 52.5 g. of sodium iodide. The reaction mixture was heated at reflux and stirred for twenty-four hours, after which time the solvent was removed by evaporation under reduced pressure. The crude product thus formed was dissolved in a mixture of 800 ml. of 1N sodium hydroxide and 1000 ml. of diethyl ether, and the mixture was stirred vigorously for forty-five minutes. The ethereal layer then was separated, washed with saturated aqueous sodium chloride and dried. Removal of the solvent by evaporation under reduced pressure afforded the product as an oil, which upon distillation provided 21.5 g. of 4a-phenyl-2-methyl-3,4,4a,5,6,7-hexahydro-2-pyrindine. B.P. 110-112°C. at 0.075 torr.

Analysis Calc. for $C_{15}H_{19}N$

Theory: C, 84.46; H, 8.98; N, 6.57.

Found: C, 84.74; H, 8.72; N, 6.28.

Example D

Following the procedure set out in Example C, 1-methyl-4-(3-methoxyphenyl)-1,2,3,6-tetrahydropyridine was reacted with 3-chloropropylbromide and sodium iodide to afford 4a-(3-methoxyphenyl)-2-methyl-3,4,4a,5,6,7-hexahydro-2-pyrindine. B.P. 132-134°C. at 0.1 torr.

Analysis Calc. for $C_{16}H_{22}NO$

Theory: C, 78.97; H, 8.70; N, 5.76.

Found: C, 76.58; H, 8.28; N, 5.36.

m/e: theory 243; found 243.

## FINAL PRODUCTS

### Example 1

A solution of 5.0 g. of 4a-phenyl-1-methyl-3,4,4a,5,6,7-hexahydro-2-pyrindine in 50 ml. of ethanol containing 500 mg of platinum oxide was stirred at room temperature for four hours under a hydrogen atomsphere of 4.13 x $10^6$ dynes/$cm^2$. The mixture then was filtered and the solvent was removed from the filtrate by evaporation to provide an oil which was shown by NMR and high pressure liquid chromatography to consist of about forty percent cis-4a-phenyl-1-methyl-2,3,4,4a,5,6,7,7a-octahydro-1H-2-pyrindine and about sixty percent of the corresponding trans isomer. The mixture was dissolved in 50 ml. of diethyl ether and made acidic by the addition of a saturated solution of hydrogen bromide dissolved in diethyl ether. Concentration of the ethereal solution effected crystallization. The mixture was filtered and the precipitate was recrystallized from 30 ml. of isopropanol and 70 ml. of diisopropyl ether to afford 2.6 g. of cis-4a-phenyl-2-methyl-2,3,4,4a,5,6,7,7a-octahydro-1H-2-pyrindinium bromide.

The filtrate was evaporated to dryness and the residue was dissolved in water. The aqueous solution was made alkaline by the addition of 1N sodium hydroxide, and the aqueous alkaline solution was extracted with diethyl ether. The ethereal extracts were combined, washed with water and dried. Removal of the solvent by evaporation under reduced

pressure afforded 2.57 g. of trans-4a-phenyl-2-methyl-2,3,4,4a,5,6,7,7a-octahydro-1H-2-pyrindine.

The trans-pyrindine derivative was dissolved in 120 ml. of ethanol and reacted with 2.76 g. of picric acid to provide 2.7 g. of trans-4a-phenyl-2-methyl-2,3,4,4a,5,6,7,7a-octahydro-1H-2-pyrindinium picrate. M.P. 167-168°C.

Analysis Calc. for $C_{21}H_{24}N_4O_7$

Theory: C, 56.75; H, 5.44; N, 12.61.

Found: C, 56.99; H, 5.65; N, 12.46.

## Example 2

The preparation described in Example 1 was repeated except that the trans-pyrindine derivative was reacted with maleic acid and isolated as trans-4a-phenyl-2-methyl-2,3,4,4a,5,6,7,7a-octahydro-1H-2-pyrindinium maleate. M.P. 113-114°C.

Analysis Calc. for $C_{19}H_{25}NO_4$

Theory: C, 68.86; H, 7.60; N, 4.23.

Found: C, 68.66; H, 7.82; N, 3.98.

## Example 3

Following the procedure set forth in Example 1, 4a-(3-methoxyphenyl)-2-methyl-3,4,4a,5,6,7-hexahydro-2-pyrindine was hydrogenated over platinum oxide to provide a 60:40 mixture of trans-4a-(3-methoxyphenyl)-2-methyl-2,3,4,4a,5,6,7,7a-octahydro-1H-2-pyrindine and the corresponding cis-isomer. The trans-isomer was crystallized as the picrate salt. The trans isomer was isolated as the free base, namely trans-4a-(3-methoxyphenyl)-2-methyl-2,3,4,4a,5,6,7,7a-octahydro-1H-2-pyrindine. M.P. 40-43°C.

Analysis Calc. for $C_{16}H_{23}NO$

Theory: C, 78.32; H, 9.45; N, 5.71.

Found: C, 78.26; H, 9.31; N, 5.61.

Example 4

A solution of 3.5 g. of trans-4a-(3-methoxyphenyl)-2-methyl-2,3,4,4a,5,6,7,7a-octahydro-1H-2-pyrindine in 35 ml. of glacial acetic acid containing 35 ml. of fifty percent aqueous hydrobromic acid was stirred and heated at reflux for fifteen hours. The reaction mixture then was cooled to room temperature and diluted with 100 ml. of ice-water. The aqueous acid solution was made basic by the addition of concentrated sodium hydroxide to pH 9.8, and the aqueous alkaline solution was extracted several times with diethyl ether. The ethereal extracts were combined, washed with water and dried. Removal of the solvent by evaporation under reduced pressure afforded 1.8 g. of the product as a solid. The solid so formed was recrystallized from 150 ml. of ethyl acetate to provide 1.65 g. of trans-4a-(3-hydroxyphenyl)-2-methyl-2,3,4,4a,5,6,7,7a-octahydro-1H-2-pyrindine. M.P. 192-194°C.

Analysis Calc. for $C_{15}H_{21}NO$

Theory: C, 77.88; H, 9.15; N, 6.05.

Found: C, 77.48; H, 8.71; N, 5.67.

CLAIMS

1. A *trans-* compound of the general formula

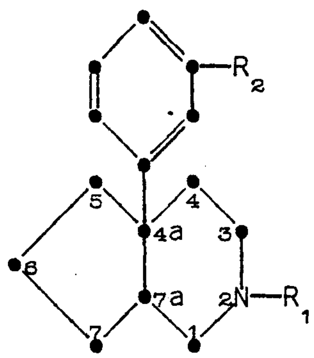

(I)

wherein:

$R_1$ is hydrogen, $C_1$-$C_8$ alkyl, $CH_2R_3$,

$-(CH_2)_n-(X)_m-$ 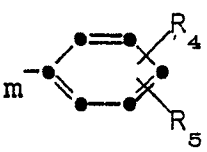 , $\overset{O}{\overset{\|}{C}}-C_1-C_7$ alkyl, $\overset{O}{\overset{\|}{C}}R_3$, or

$\overset{O}{\overset{\|}{C}}-(CH_2)_{n-1}-(X)_m-$ 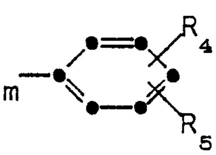 ;

in which:

$R_3$ is $C_2$-$C_7$ alkenyl, $C_3$-$C_6$ cycloalkyl, furyl, or tetrahydrofuryl;

$R_4$ and $R_5$ independently are hydrogen, $C_1$-$C_3$ alkyl, or halogen;

n is 0, 1, 2, or 3;

m is 0 or 1, except that when m is 0, n is other than 0;

X is CO, CHOH, CH=CH, S, or O, except that when n is O, X is other than S or O;

$R_2$ is hydrogen, hydroxy, $C_1$-$C_3$ alkoxy, or $C_1$-$C_3$ alkanoyloxy; and

the non-toxic pharmaceutically acceptable acid addition salts thereof.

2. A compound of claim 1 wherein $R_1$ is $C_1$-$C_8$ alkyl or $CH_2R_3$ in which $R_3$ is $C_2$-$C_7$ alkenyl or $C_3$-$C_6$ cycloalkyl.

3. A compound of claim 2 wherein $R_2$ is hydroxy or $C_1$-$C_3$ alkoxy.

4. A compound of claim 3 wherein $R_2$ is methoxy.

5. A compound of claim 3 wherein $R_2$ is hydroxy.

6. A compound of claim 1 wherein $R_1$ is hydrogen.

7. A compound of claim 1 wherein $R_1$ is $C_1$-$C_8$ alkyl.

8. Any one of the following compounds of claim 1 or its pharmaceutically acceptable salt:

Trans-4a-phenyl-2-methyl-2,3,4,4a,5,6,7,7a-octahydro-1H-pyrindine,

Trans-4a-(3-methoxyphenyl)-2-methyl-2,3,4,4a,5,6,7,7a-octahydro-1H-2-pyrindine,

Trans-4a-(3-hydroxyphenyl)-2-methyl-2,3,4,4a,5,6,7,7a-octahydro-1H-2-pyrindine.

9. A process for preparing a <u>trans</u>-compound of the general formula

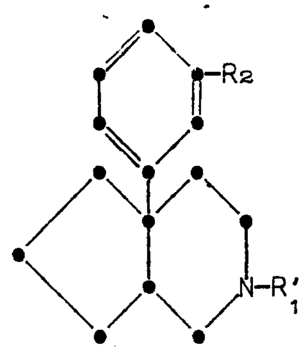

(II)

wherein:

$R_1'$ is hydrogen, $C_1$-$C_8$ alkyl, $CH_2R_3$, or

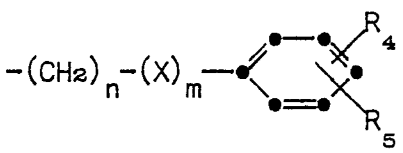

;

in which:

$R_3$ is $C_2$-$C_7$ alkenyl, $C_3$-$C_6$ cycloalkyl, furyl, or tetrahydrofuryl;

$R_4$ and $R_5$ independently are hydrogen, $C_1$-$C_3$ alkyl, or halogen;

n is 0, 1, 2, or 3;

m is 0 or 1, except that when m is 0, n is other than 0;

X is CO, CHOH, CH=CH, S, or O, except that when n is 0, X is other than S or O;

$R_2$ is hydrogen, hydroxy, $C_1$-$C_3$ alkoxy, or $C_1$-$C_3$ alkanoyloxy; and

the pharmaceutically acceptable acid addition salts thereof;

which comprises reacting a compound of the general formula

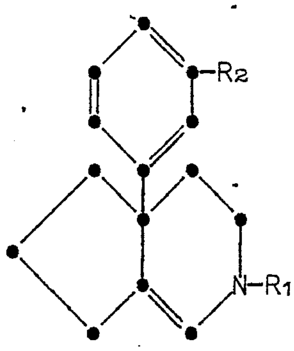

(III)

wherein:

$R_1$ is hydrogen, $C_1$-$C_8$ alkyl, $CH_2R_3'$,

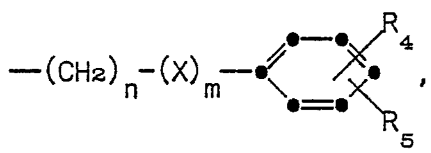, 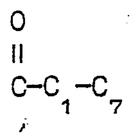 alkyl, 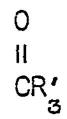, or

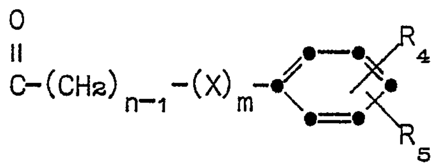

in which:

$R_3'$ is $C_3$-$C_6$ cycloalkyl, furyl, or tetrahydrofuryl;

$R_4$ and $R_5$ independently are hydrogen, $C_1$-$C_3$ alkyl, or halogen;

n is 0, 1, 2, or 3;

m is 0 or 1, except that when m is 0, n is other than 0;

X is CO, CHOH, CH=CH, S, or O, except that when n is 0, X is other than S or O;

$R_2$ is hydrogen, hydroxy, $C_1$-$C_3$ alkoxy, or $C_1$-$C_3$ alkanoyloxy;

with hydrogen and platinum oxide, followed by alkylation when $R_1$ is hydrogen to obtain a compound of formula (II) in which $R_1'$ is defined as before, optionally de-etherifying when $R_2$ is $C_1$-$C_3$ alkoxy to obtain a compound of formula (II) in which $R_2$ is hydroxy, and optionally acylating a compound of formula (II) in which $R_2$ is hydroxy to obtain a compound of formula (II) in which $R_2$ is $C_1$-$C_3$ alkanoyloxy, and, when desired, forming the pharmaceutically acceptable acid addition salt thereof by conventional means.

10. A pharmaceutical formulation comprising as an active ingredient a trans-compound of the general formula

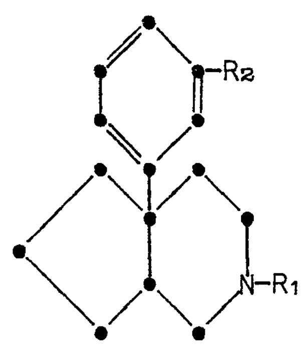

(I)

wherein the various symbols are defined as in claim 1, or a pharmaceutically-acceptable acid addition salt thereof, associated with a pharmaceutically-acceptable carrier therefor.

11. A compound as defined in any of claims 1 to 8 or a pharmaceutically-acceptable acid addition salt thereof, for use as a pharmaceutical.

12. A compound as defined in any of claims 1 to 8, or a pharmaceutically-acceptable acid addition salt thereof, for use as an analgesic agent.

European Patent Office

**EUROPEAN SEARCH REPORT**

0002937

Application number

EP 78 30 0879

## DOCUMENTS CONSIDERED TO BE RELEVANT

| Category | Citation of document with indication, where appropriate, of relevant passages | Relevant to claim |
|---|---|---|
| D | FR - A - 2 193 600 (DU PONT DE NEMOURS)<br>* Claims 1-3, 20-21 *<br>& BE - A - 802 557<br>-- | 1-7, 10-12 |
| P | FR - A - 2 369 267 (ELI LILLY) May 26th, 1978<br>* Claims 1-7,9; page 19, lines 6-22 *<br>---- | 1-7, 9-12 |

CLASSIFICATION OF THE APPLICATION (Int. Cl.²)

C 07 D 221/04
405/06
A 61 K 31/435//
C 07 D 211/52
211/70

TECHNICAL FIELDS SEARCHED (Int.Cl.²)

C 07 D 221/04
A 61 K 31/435
C 07 D 405/06

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 27-3-1979 | NUYTS |